# EUROPEAN PATENT APPLICATION

(11) **EP 4 654 794 A1**
(43) Date of publication of application: **26.11.2025**
(21) Application number: 24774274.5
(22) Date of filing: 22.03.2024
(51) Int. Cl.: H10K 50/14

(54) **LIGHT-EMITTING SUBSTRATE AND LIGHT-EMITTING APPARATUS**

(30) Priority: 23.03.2023 WO PCT/CN2023/083509
(71) Applicant: BOE Technology Group Co., Ltd., Beijing 100015 (CN); Beijing BOE Technology Development Co., Ltd., Beijing 100176 (CN)
(72) Inventor: CHEN, Zhuo, Beijing 100176 (CN); LI, Dong, Beijing 100176 (CN); LU, Shaoyong, Beijing 100176 (CN)
(74) Representative: Hoffmann Eitle
(86) International application number: PCT/CN2024/083427
(87) International publication number: WO 2024/193711

(57) **Abstract**

A light-emitting substrate. The light-emitting substrate comprises: a substrate and a plurality of light-emitting devices, the plurality of light-emitting devices being arranged on the substrate. Each light-emitting device among the plurality of light-emitting devices comprises: a first electrode, a second electrode and a light-emitting pattern provided between the first electrode and the second electrode, the first electrode being closer to the substrate than the second electrode. The plurality of light-emitting devices comprise: at least one first light-emitting device. The at least one first light-emitting device comprises: a first carrier transport layer, the first carrier transport layer being arranged on the side of the light-emitting pattern comprised in the at least one first light-emitting device close to the substrate and being in contact with the light-emitting pattern comprised in the at least one first light-emitting device, and the material of at least one of the light-emitting pattern comprised in the at least one first light-emitting device and the first carrier transport layer comprising a cross-linking material.

## Description

This application claims priority to PCT Patent Application No. PCT/CN2023/083509, filed on March 23, 2023, which is incorporated herein by reference in its entirety.

### TECHNICAL FIELD

The present disclosure relates to the field of display technologies, and in particular, to a light-emitting substrate and a light-emitting apparatus.

### BACKGROUND

As a new type of light-emitting material, quantum dot (QD) has the advantages of high light color purity, high luminous quantum efficiency, adjustable light-emitting color, and long service life, which makes it a hot spot in the research of new LED (light-emitting diode) light-emitting materials. Therefore, a quantum dot light-emitting diode (QLED) with quantum dots as its light-emitting layer has become the main direction of the current research on new display apparatuses.

### SUMMARY

In an aspect, a light-emitting substrate is provided. The light-emitting substrate includes a base and a plurality of light-emitting devices. The plurality of light-emitting devices are provided on the base. Each light-emitting device in the plurality of light-emitting devices includes a first electrode, a second electrode, and a light-emitting pattern provided between the first electrode and the second electrode, the first electrode being closer to the base than the second electrode.

The plurality of light-emitting devices include at least one first light-emitting device. The at least one first light-emitting device includes a first carrier transport layer, the first carrier transport layer is provided on a side of the light-emitting pattern contained in the at least one first light-emitting device proximate to the base and in contact with the light-emitting pattern contained in the at least one first light-emitting device. A material of at least one of the light-emitting pattern contained in the at least one first light-emitting device and the first carrier transport layer includes a cross-linked material.

In some embodiments, the first carrier transport layer includes an electron transport layer, a hole transport layer, and a hole injection layer.

In some embodiments, the electron transport layer and the light-emitting pattern contained in the at least one first light-emitting device are provided therebetween with an auxiliary layer, and a material of the auxiliary layer is the same as a material of one layer in the first carrier transport layer.

In some embodiments, the material of the auxiliary layer is the same as the material of the hole transport layer, and the material of the auxiliary layer is a first cross-linked material, the first cross-linked material being generated by cross-linking of a hole transport material and a first photosensitive material under light radiation.

In some embodiments, the first cross-linked material is selected from any one of structures shown in following general formula VI: 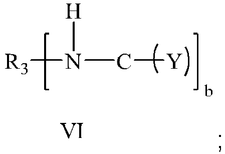 where R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; b is a positive integer taking a value greater than or equal to 2; and Y represents a remaining structure of a first carrier transport material with a C-H bond removed, the C-H bond is used for undergoing a cross-linking reaction with a first photosensitive group of the first photosensitive material under light radiation.

In some embodiments, the first cross-linked material is selected from any one of structures shown in following general formula VII: 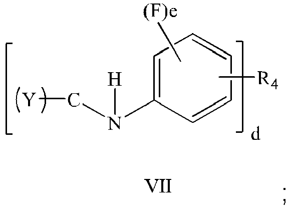 where R₄ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; d is a positive integer taking a value greater than or equal to 2; e takes any one of values 0, 1, 2, 3 and 4; and Y represents a remaining structure of a first carrier transport material with a C-H bond removed, the C-H bond is used for undergoing a cross-linking reaction with a first photosensitive group of the first photosensitive material.

In some embodiments, the first cross-linked material is selected from any one of structures shown in following general formula XI-A: 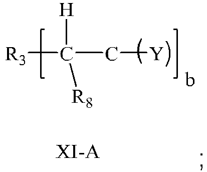 where R₈ and R₃ are each independently selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; Y represents a remaining structure of a first carrier transport material with a C-H bond removed, the C-H bond is used for undergoing a cross-linking reaction with a first photosensitive group of the first photosensitive material; and b is a positive integer taking a value greater than or equal to 2.

In some embodiments, the first photosensitive material includes the first photosensitive group; and the first photosensitive group includes at least one of an azide group, a diazirine group and a diazo group.

In some embodiments, the material of the auxiliary layer is the same as the material of the hole transport layer, and the material of the auxiliary layer is a first material without cross-linking.

In some embodiments, the first material includes a hole transport material, or includes the hole transport material and a first photosensitive material.

In some embodiments, a material of the light-emitting pattern contained in the first light-emitting device includes a first cross-linked quantum dot material, the first cross-linked quantum dot material being generated by cross-linking a first quantum dot material and a second photosensitive material under light radiation.

In some embodiments, the first cross-linked quantum dot material is selected from any one of structures shown in following general formula IV: 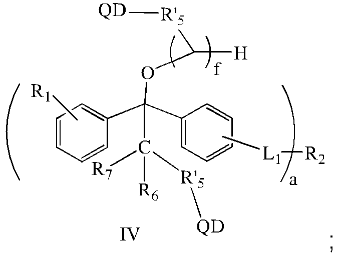 where L₁ is selected from any one of a single bond, an ester bond, an ether bond, and a thioether bond; R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, where in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃; R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain with an amino group, and a C1 to C40 carbon chain with a sulfhydryl group; and any one of the carboxyl group, the amino group, and the sulfhydryl group is connected to the quantum dot body by coordination; and a is a positive integer taking a value greater than or equal to 1, and f is a positive integer taking a value greater than or equal to 1.

In some embodiments, the first cross-linked quantum dot material is selected from any one of structures shown in following general formula V: 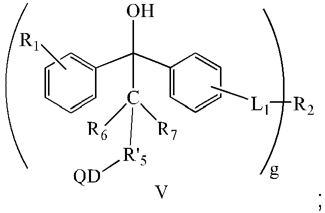 where L₁ is selected from any one of a single bond, an ester bond, an ether bond, and a thioether bond; R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, where in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃; R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination; and g is a positive integer taking a value greater than or equal to 2.

In some embodiments, the first cross-linked quantum dot material is selected from any one of structures shown in following general formula VIII: 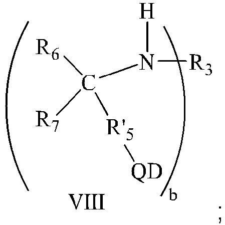 where QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, where in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃; R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination; R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; and b is a positive integer taking a value greater than or equal to 2.

In some embodiments, the first cross-linked quantum dot material is selected from any one of structures shown in following general formula IX: 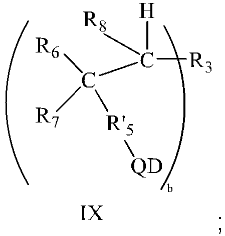 where QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, where in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbI₃; R₃ and R₈ are each independently selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination; R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; and b is a positive integer taking a value greater than or equal to 2.

In some embodiments, the first quantum dot material includes a quantum dot body and a first ligand material coordinated on the quantum dot body; and the first ligand material contains a C-H bond, and/or the first ligand material contains a double bond.

In some embodiments, the second photosensitive material includes a second photosensitive group, and the second photosensitive group includes a benzophenone group.

In some embodiments, the plurality of light-emitting devices further include at least one second light-emitting device and at least one third light-emitting device. The at least one first light-emitting device is configured to emit light of first wavelength, the light of first wavelength being red light; the at least one second light-emitting device is configured to emit light of second wavelength, the light of second wavelength being green light; and the at least one third light-emitting device is configured to emit light of third wavelength, the light of third wavelength being blue light.

In some embodiments, the light-emitting device includes multiple light-emitting patterns arranged in a stack, with a charge generation layer being provided between every two adjacent light-emitting patterns in the multiple light-emitting patterns.

In another aspect, a light-emitting apparatus is provided. The light-emitting apparatus includes the light-emitting substrate according to any of the above embodiments.

### BRIEF DESCRIPTION OF THE DRAWINGS

In order to describe technical solutions in some embodiments of the present disclosure more clearly, the accompanying drawings to be used in some embodiments of the present disclosure will be introduced briefly; obviously, the accompanying drawings to be described below are merely some embodiments of the present disclosure, and a person of ordinary skill in the art can obtain other drawings according to those drawings. In addition, the accompanying drawings in the following description may be regarded as schematic diagrams, but are not limitations on actual sizes of products, actual processes of methods and actual timings of signals involved in the embodiments of the present disclosure.
FIG. 1 is a flowchart of manufacturing a light-emitting substrate, provided in accordance with some embodiments;
FIG. 2 is a structural diagram of a light-emitting substrate, provided in accordance with some embodiments;
FIG. 3 is a structural diagram of a light-emitting device, provided in accordance with some embodiments;
FIG. 4 is a curve graph showing the variation of current density with voltage for a light-emitting device, provided in accordance with some embodiments of the present disclosure;
FIG. 5 is a structural diagram of a light-emitting device, provided in accordance with some embodiments of the present disclosure;
FIG. 6 is a structural diagram of another light-emitting device, provided in accordance with some embodiments of the present disclosure;
FIG. 7 is a structural diagram of yet another light-emitting device, provided in accordance with some embodiments of the present disclosure;
FIG. 8 is a curve graph showing the variation of external quantum efficiency with voltage for a light-emitting device, provided in accordance with some embodiments of the present disclosure;
FIG. 9 is a structural diagram of still yet another light-emitting device, provided in accordance with some embodiments of the present disclosure;
FIG. 10 is a structural diagram of still yet another light-emitting device, provided in accordance with some embodiments of the present disclosure;
FIG. 11 is a structural diagram of still yet another light-emitting device, provided in accordance with some embodiments of the present disclosure;
FIG. 12 is a curve graph showing the variation of current density with voltage for a light-emitting device, provided in accordance with some embodiments of the present disclosure;
FIG. 13 is a curve graph showing the variation of light absorption intensity with wavelength for a light-emitting device, provided in accordance with some embodiments of the present disclosure;
FIG. 14 is a structural diagram showing the cross-linking of a second photosensitive material with a quantum dot material, provided in accordance with some embodiments of the present disclosure;
FIG. 15 is another structural diagram showing the cross-linking of a second photosensitive material with a quantum dot material, provided in accordance with some embodiments of the present disclosure;
FIG. 16 is a structural diagram showing the cross-linking of a first photosensitive material with a carrier transport material, provided in accordance with some embodiments of the present disclosure;
FIG. 17 is another structural diagram showing the cross-linking of a first photosensitive material with a carrier transport material, provided in accordance with some embodiments of the present disclosure;
FIG. 18 is a curve graph showing the variation of current efficiency with voltage for another light-emitting device, provided in accordance with some embodiments of the present disclosure;
FIG. 19 is a curve graph showing the variation of current efficiency with voltage for yet another light-emitting device, provided in accordance with some embodiments of the present disclosure;
FIG. 20 is a structural diagram of still yet another light-emitting device, provided in accordance with some embodiments of the present disclosure;
FIG. 21 is a curve graph showing the variation of current efficiency with voltage for still yet another light-emitting device, provided in accordance with some embodiments of the present disclosure;
FIG. 22 is a structural diagram of still yet another light-emitting device, provided in accordance with some embodiments of the present disclosure;
FIG. 23 is a structural diagram of a light-emitting apparatus, provided in accordance with some embodiments of the present disclosure;
FIG. 24 is a curve graph showing the variation of current efficiency with voltage for a light-emitting device, provided in accordance with some embodiments of the present disclosure;
FIG. 25 is a flowchart of manufacturing a light-emitting substrate, provided in accordance with some embodiments of the present disclosure; and
FIG. 26 is a structural diagram of still yet another light-emitting device, provided in accordance with some embodiments of the present disclosure.

### DETAILED DESCRIPTION

The technical solutions in some embodiments of the present disclosure will be described clearly and completely with reference to the accompanying drawings; obviously, the described embodiments are merely some but not all embodiments of the present disclosure. All other embodiments obtained by a person of ordinary skill in the art based on embodiments of the present disclosure shall be included in the protection scope of the present disclosure.

Unless the context requires otherwise, throughout the specification and the claims, the term "comprise" and other forms thereof such as the third-person singular form "comprises" and the present participle form "comprising" are construed as an open and inclusive meaning, i.e., "including, but not limited to." In the description of the specification, the terms such as "one embodiment," "some embodiments," "exemplary embodiments," "example," "specific example," or "some examples" are intended to indicate that specific features, structures, materials, or characteristics related to the embodiment(s) or example(s) are included in at least one embodiment or example of the present disclosure. Schematic representations of the above terms do not necessarily refer to the same embodiment(s) or example(s). In addition, the specific features, structures, materials, or characteristics may be included in any one or more embodiments or examples in any suitable manner.

Hereinafter, the terms "first" and "second" are only used for descriptive purposes, and are not to be construed as indicating or implying the relative importance or implicitly indicating the number of indicated technical features. Thus, features defined by "first" or "second" may explicitly or implicitly include one or more of the features. In the description of the embodiments of the present disclosure, the term "a plurality of" or "multiple" means two or more unless otherwise specified.

The phrase "at least one of A, B and C" has the same meaning as the phrase "at least one of A, B or C," and they both include the following combinations of A, B and C: only A, only B, only C, a combination of A and B, a combination of A and C, a combination of B and C, and a combination of A, B and C.

The phrase "A and/or B" includes the following three combinations: only A, only B, and a combination of A and B.

The term such as "about," "substantially" or "approximately" as used herein includes a stated value and an average value within an acceptable range of deviation of a particular value determined by a person of ordinary skill in the art, considering measurement in question and errors associated with measurement of a particular quantity (i.e., limitations of a measurement system).

The term such as "parallel," "perpendicular," or "equal" as used herein includes a stated condition and a condition similar to the stated condition. A range of the similar condition is within an acceptable deviation range, and the acceptable deviation range is determined by a person of ordinary skill in the art, considering measurement in question and errors associated with measurement of a particular quantity (i.e., the limitations of a measurement system). For example, the term "parallel" includes absolute parallelism and approximate parallelism, and an acceptable range of deviation of the approximate parallelism may be a deviation within 5°; the term "perpendicular" includes absolute perpendicularity and approximate perpendicularity, and an acceptable range of deviation of the approximate perpendicularity may also be a deviation within 5°; and the term "equal" includes absolute equality and approximate equality, and an acceptable range of deviation of the approximate equality may be a difference between two equals being less than or equal to 5% of either of the two equals.

It should be understood that, when a layer or element is referred to as being on another layer or substrate, it may be that the layer or element is directly on the another layer or substrate, or it may be that intervening layer(s) exist between the layer or element and the another layer or substrate.

Exemplary embodiments are described in the present disclosure with reference to sectional views and/or plan views as idealized exemplary drawings. In the accompanying drawings, thicknesses of layers and sizes of regions/areas are enlarged for clarity. Thus, variations in shape with respect to the accompanying drawings due to, for example, manufacturing technologies and/or tolerances may be envisaged. Therefore, the exemplary embodiments should not be construed as being limited to the shapes of the regions shown herein, but including shape deviations due to, for example, manufacturing. For example, an etched region shown to have a rectangular shape generally has a curved feature. Therefore, the regions shown in the accompanying drawings are schematic in nature, and their shapes are not intended to show actual shapes of the regions in a device, and are not intended to limit the scope of the exemplary embodiments.

The quantum dot light-emitting diode (QLED) with quantum dots (QD) as a light-emitting material is widely used in the display field. The manufacture techniques for a light-emitting layer of the quantum dot light-emitting diode mainly include inkjet printing technology, photolithography, transfer technology, etc., in which the photolithography is one of the more promising methods for manufacturing high-resolution quantum dot light-emitting diodes. The present disclosure is proposed by way of example of a quantum dot light-emitting device and a light-emitting device manufactured by the photolithography.

The photolithography is a technology for patterning quantum dots by means of exposure and development.

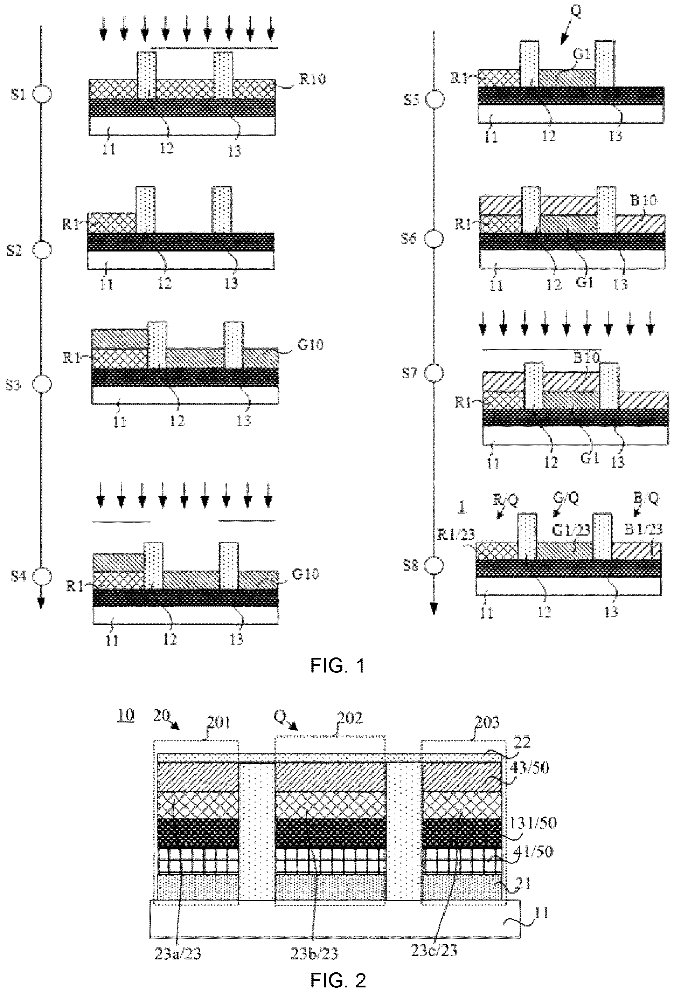

As shown in FIG. 1, a process for forming light-emitting patterns 23 of a light-emitting substrate 1 is described as illustrated. Referring to the last sub-figure in FIG. 1 (i.e., the sub-figure corresponding to a step S8), the light-emitting substrate 1 includes a base 11, and a pixel defining layer 12 and a plurality of light-emitting devices that are provided on the base 11, in which each light-emitting device includes a light-emitting pattern 23. Here, the pixel defining layer 12 has a plurality of openings Q, and the plurality of light-emitting devices may be provided in one-to-one correspondence with the plurality of openings Q.

For example, the plurality of light-emitting devices include red light-emitting devices R, green light-emitting devices G, and blue light-emitting devices B. The following describes a process for sequentially forming a light-emitting pattern R1 of the red light-emitting device R, a light-emitting pattern G1 of the green light-emitting device G, and a light-emitting pattern B1 of the blue light-emitting device B.

As shown in FIG. 1, the process includes steps S1 to S8.

In order to improve the efficiency of injecting electrons and holes into the light-emitting pattern 23, there is further provided a front film layer 13 between the base 11 and the light-emitting pattern 23. For example, the front film layer 13 is an electron transport layer or a hole transport layer.

In S1, a side of the front film layer 13 away from the base 11 is coated with a red quantum dot (RQD) light-emitting material to form a first initial light-emitting pattern R10, and the first initial light-emitting pattern R10 is exposed, with a region where the red light-emitting device R is to be formed being an exposed region.

In S2, the first initial light-emitting pattern R10 is developed, with an exposed region of the first initial light-emitting pattern R10 being retained, to form the light-emitting pattern R1 of the red light-emitting device R.

In S3, a side of the light-emitting pattern R1 of the red light-emitting device R away from the base 11 is coated with a green quantum dot (GQD) light-emitting material to form a second initial light-emitting pattern G10.

In S4, the second initial light-emitting pattern G10 is exposed, with a region where the green light-emitting device G is to be formed being an exposed region.

In S5, the second initial light-emitting pattern G10 is developed, with an exposed region of the second initial light-emitting pattern G10 being retained, to form the light-emitting pattern G1 of the green light-emitting device G.

In S6, a side of the light-emitting pattern G1 of the green light-emitting device G away from the base 11 is coated with a blue quantum dot (BQD) light-emitting material to form a third initial light-emitting pattern B10.

In S7, the third initial light-emitting pattern B10 is exposed, with a region where the blue light-emitting device B is to be formed being an exposed region.

In S8, the third initial light-emitting pattern B10 is developed, with an exposed region of the third initial light-emitting pattern B10 being retained, to form the light-emitting pattern B1 of the blue light-emitting device B.

However, the inventors have found that in light-emitting devices of quantum dots formed by using lithography, there exist problems such as imbalance in the carrier transport rates of electrons and holes in the light-emitting devices, low device light-emitting efficiency, and different performances of different monochromatic light-emitting devices. The present disclosure aims to solve at least one of the above problems.

In light of the above issues, as shown in FIG. 2, some embodiments of the present disclosure provide a light-emitting substrate 10. The light-emitting substrate 10 includes a base 11 and a plurality of light-emitting devices 20. The plurality of light-emitting devices 20 are provided on the base 11. Each light-emitting device 20 in the plurality of light-emitting devices 20 includes a first electrode 21, a second electrode 22, and a light-emitting pattern 23 provided between the first electrode 21 and the second electrode 22, where the first electrode 21 is closer to the base 11 than the second electrode 22.

For example, as shown in FIG. 2, the light-emitting substrate 10 further includes a pixel defining layer 12 provided on the base 11. The pixel defining layer 12 has a plurality of openings Q, and the plurality of light-emitting devices 20 may be provided in one-to-one correspondence with the plurality of openings Q.

For example, the base 11 may be made of an inorganic material, an organic material, a silicon wafer, or a composite material layer.

Examples of the inorganic material may be glass, metal, and the like. Examples of the organic material may be polycarbonate, polymethylmethacrylate, polyethylene terephthalate, polyethylene glycol naphthalate, polyamide, polyether sulfone, combinations thereof, and the like.

In some examples, the first electrode 21 may be an anode, and the second electrode 22 is a cathode in this case. In some other examples, the first electrode 21 may be a cathode, and the second electrode 22 is an anode in this case.

In a case where the first electrode 21 is an anode and the second electrode 22 is a cathode, the light-emitting principle of the light-emitting device 20 is as follows: by means of a circuit that the anode and the cathode are connected to, holes from the anode are injected into the light-emitting pattern 23 and electrons from the cathode are injected into the light-emitting pattern 23, the electrons and the holes form excitons in the light-emitting pattern 23, and the excitons return to the ground state by radiative transitions to emit photons.

As shown in FIG. 2, the plurality of light-emitting devices 20 include at least one first light-emitting device 201. The at least one first light-emitting device 201 includes a first carrier transport layer 50. The first carrier transport layer 50 includes an electron transport layer 43, a hole transport layer 131, and a hole injection layer 41.

Here, a material of at least one of a light-emitting pattern 23a contained in the at least one first light-emitting device 201 and the first carrier transport layer 50 includes a cross-linked material.

For example, the first light-emitting device 201 may be a light-emitting device for emitting red light, and a first quantum dot material may be a red quantum dot light-emitting material.

The solubility of the cross-linked material in a developing solution is relatively small, that is, the cross-linked material has a relatively good resistance to solvent immersion, so a film layer formed by the cross-linked material is not easy to be dissolved, so as to make the film layer of the light-emitting device 20 have a good stability, which is conducive to improving the stability of the light-emitting device 20.

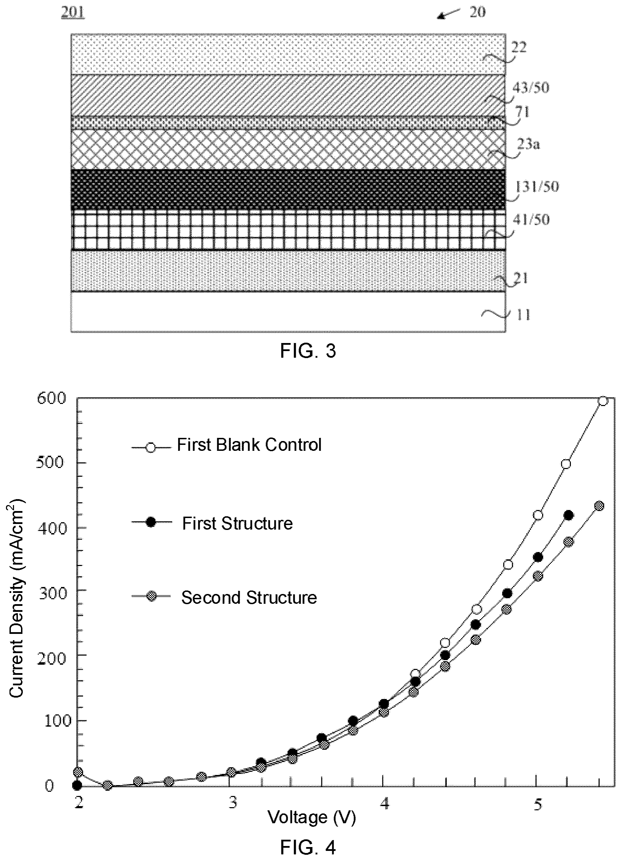

In some embodiments, as shown in FIG. 3, the plurality of light-emitting devices 20 further include at least one second light-emitting device 202 and at least one third light-emitting device 203.

For example, the at least one first light-emitting device 201 is configured to emit light of first wavelength, e.g., the light of first wavelength is red light. The at least one second light-emitting device 202 is configured to emit light of second wavelength, e.g., the light of second wavelength is green light. The at least one third light-emitting device 203 is configured to emit light of third wavelength, e.g., the light of third wavelength is blue light.

The following describes a film layer structure of the light-emitting substrate 10 in terms of a film layer configuration of a light-emitting device 20, in which the first light-emitting device 201, the second light-emitting device 202, and the third light-emitting device 203 may have the same or different film layer configurations, which are not limited herein.

In some embodiments, as shown in FIG. 3, the electron transport layer 43 and the light-emitting pattern 23a contained in the at least one first light-emitting device 201 are provided therebetween with an auxiliary layer 71. A material of the auxiliary layer 71 is the same as a material of one layer in the first carrier transport layer 50.

For example, the material of the auxiliary layer 71 is the same as the material of the hole transport layer 131, and the material of the auxiliary layer 71 is a first cross-linked material, where the first cross-linked material is generated by cross-linking of a hole transport material and a first photosensitive material under light radiation.

For example, the material of the auxiliary layer 71 is the same as the material of the hole transport layer 131, and the material of the auxiliary layer 71 is a first material without cross-linking. For example, the first material includes a hole transport material and a first photosensitive material.

For the descriptions of the first photosensitive material and the first cross-linked material, please refer to the subsequent contents, which will not be described in detail here.

The auxiliary layer 71 can serve as an electron blocking layer of the first light-emitting device 201 for improving the electron injection of the first light-emitting device 201 and enhancing the efficiency of the light-emitting device 20.

Based on the above specific implementations, the following experimental data is provided to demonstrate that the auxiliary layer 71 has the effect of blocking the injection of electrons into the light-emitting pattern 23 and regulating the carrier transport rates of electrons and holes.

By way of example, as shown in FIG. 4, the horizontal coordinate of the graph represents the voltage (in units of V) and the vertical coordinate represents the current density (in units of mA/cm²) of the first light-emitting device 201. This example provides curves showing the variation of the current density with the voltage for three types of first light-emitting devices 201.

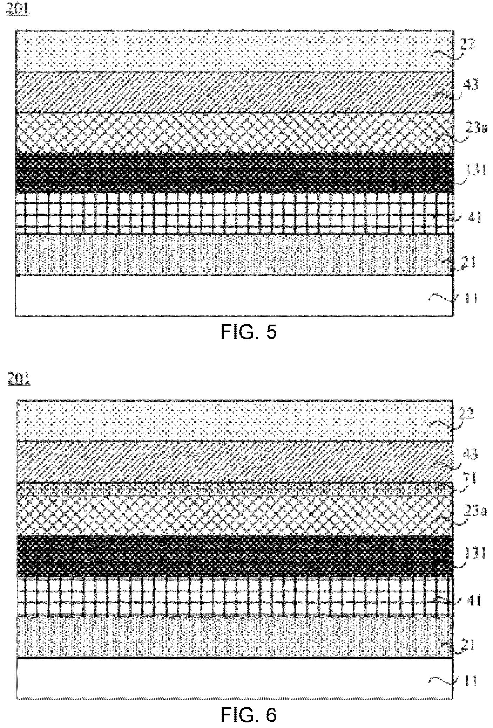

Here, as shown in FIG. 5, the first light-emitting device 201 includes a first electrode 21, a hole injection layer 41, a hole transport layer 131, a light-emitting pattern 23a, an electron transport layer 43, and a second electrode 22, which are arranged in a stack on the base 11 in sequence.

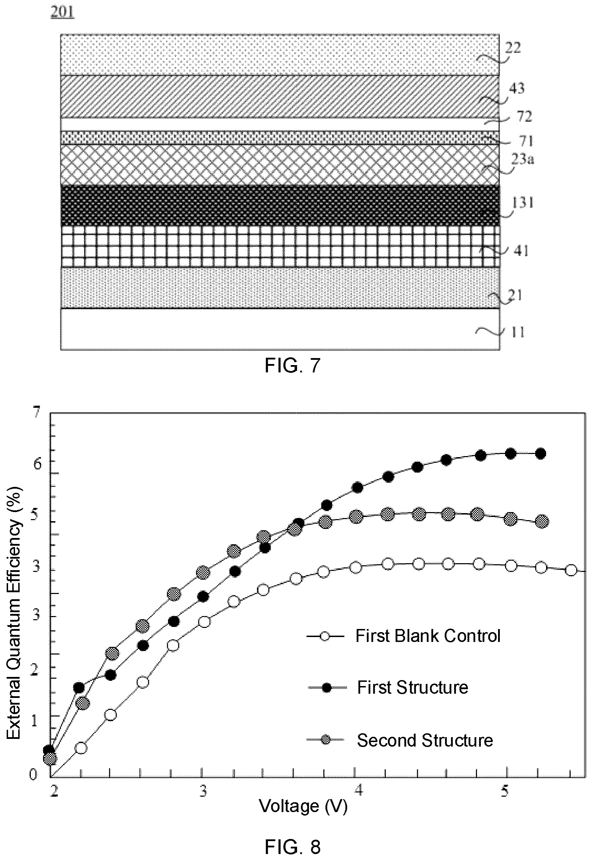

As shown in FIG. 4 and FIG. 5, "First Blank Control" refers to a configuration in which an electron transport layer 43 is formed on a side of a light-emitting pattern 23a away from the base 11; as shown in FIG. 4 and FIG. 6, "First Structure" refers to a configuration in which an auxiliary layer 71 is provided on a side of a light-emitting pattern 23a away from the base 11, and an electron transport layer 43 is provided on a side of the auxiliary layer 71 away from the base 11; and as shown in FIG. 4 and FIG. 7, "Second Structure" refers to a configuration in which an auxiliary layer 71 is provided on a side of a light-emitting pattern 23a away from the base 11, another auxiliary layer 72 is provided on a side of the auxiliary layer 71 away from the base 11, and an electron transport layer 43 is provided on a side of the auxiliary layer 72 away from the base 11.

As can be seen in FIG. 4, current densities corresponding to the curve of "First Structure" and the curve of "Second Structure" are reduced, indicating that at least one auxiliary layer 71 has the effect of blocking electrons. Therefore, the auxiliary layer 71 can serve as an electron blocking layer of the first light-emitting device 201.

Furthermore, as shown in FIG. 8, the horizontal coordinate represents the voltage (in units of V), and the vertical coordinate represents the external quantum efficiency (EQE), in which the structures of the first light-emitting devices 201 represented by "First Blank Control," "First Structure" and "Second Structure" can be referred to as described above, and will not be repeated herein.

As can be seen from FIG. 8, external quantum efficiencies corresponding to the curve of "First Structure" and the curve of "Second Structure" are improved, indicating that the provision of at least one auxiliary layer 71 can make the transport of carriers (including electrons and holes) of the first light-emitting device 201 more balanced, which is conducive to improving the external quantum efficiency of the first light-emitting device 201.

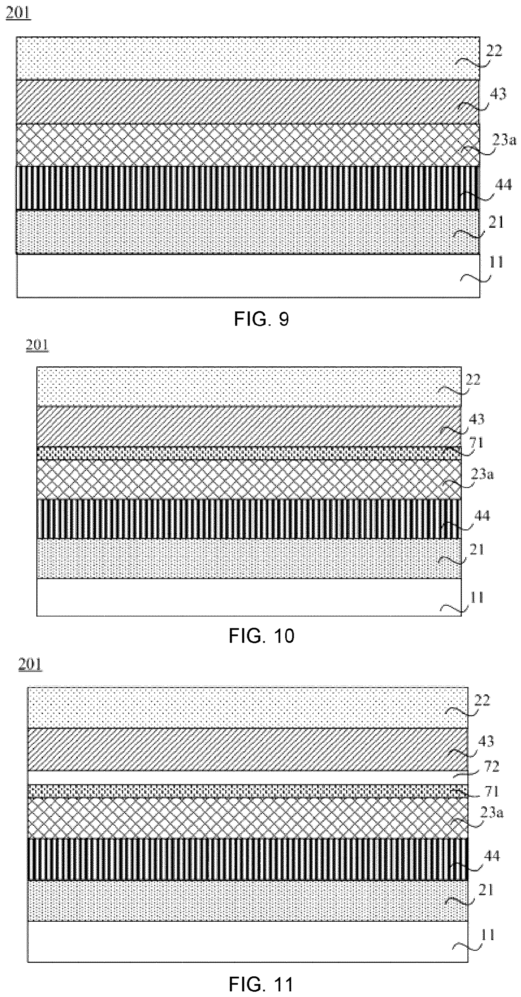

By way of example, as shown in FIG. 9 to FIG. 12, it is also possible to demonstrate that the provision of the auxiliary layer 71 can improve the external quantum efficiency of the light-emitting device 20 in a case where the hole injection layer 41 and the hole transport layer 131 are not provided between the light-emitting pattern 23a and the first electrode 21, but only the hole blocking layer 44 is provided therebetween.

As shown in FIG. 9, the first light-emitting device 201 includes a first electrode 21, a hole blocking layer 44, a light-emitting pattern 23a, an electron transport layer 43, and a second electrode 22, which are arranged in a stack on the base 11 in sequence.

As shown in FIG. 10, the first light-emitting device 201 includes a first electrode 21, a hole blocking layer 44, a light-emitting pattern 23a, an auxiliary layer 71, an electron transport layer 43, and a second electrode 22, which are arranged in a stack on the base 11 in sequence.

As shown in FIG. 11, the first light-emitting device 201 includes a first electrode 21, a hole blocking layer 44, a light-emitting pattern 23a, an auxiliary layer 71, another auxiliary layer 72, an electron transport layer 43, and a second electrode 22, which are arranged in a stack on the base 11 in sequence.

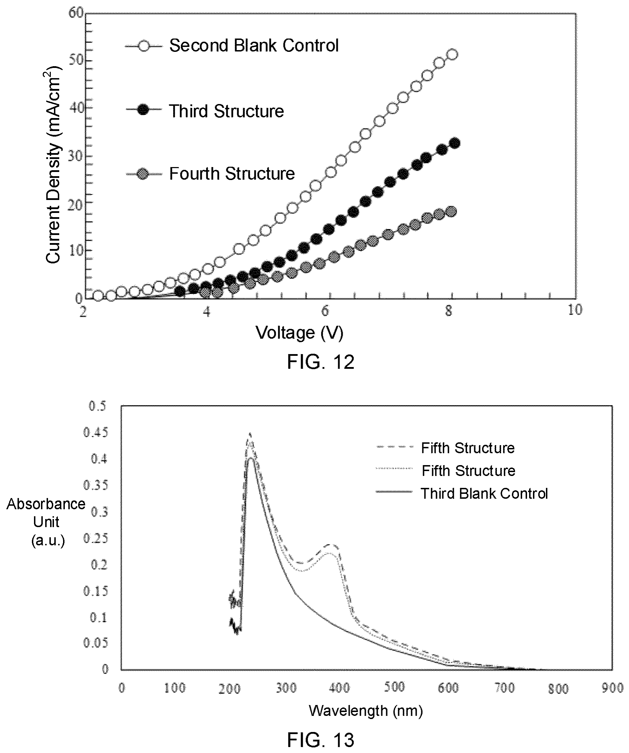

As shown in FIG. 12, the horizontal coordinate represents the voltage (in units of V), and the vertical coordinate represents the current density (in units of mA/cm²) of the light-emitting device 20.

Here, as shown in FIG. 9 and FIG. 12, "Second Blank Control" refers to a configuration in which an electron transport layer 43 is formed on a side of a light-emitting pattern 23a away from the base 11; as shown in FIG. 10 and FIG. 12, "Third Structure" refers to a configuration in which an auxiliary layer 71 is provided on a side of a light-emitting pattern 23a away from the base 11, and an electron transport layer 43 is provided on a side of the auxiliary layer 71 away from the base 11; and as shown in FIG. 11 and FIG. 12, "Fourth Structure" refers to a configuration in which an auxiliary layer 71 is provided on a side of a light-emitting pattern 23a away from the base 11, another auxiliary layer 72 is provided on a side of the auxiliary layer 71 away from the base 11, and an electron transport layer 43 is provided on a side of the auxiliary layer 72 away from the base 11.

As can be seen in FIG. 12, current densities corresponding to the curve of "Third Structure" and the curve of "Fourth Structure" are reduced, indicating that at least one auxiliary layer 71 has the effect of blocking electrons, which is beneficial to improving the external quantum efficiency of the first light-emitting device 201.

For example, the first light-emitting device 201 may be a light-emitting device for emitting red light, and a first quantum dot material may be a red quantum dot light-emitting material. For example, the second light-emitting device 202 may be a light-emitting device for emitting green light, and a second quantum dot material may be a green quantum dot light-emitting material. For example, the third light-emitting device 203 may be a light-emitting device for emitting blue light, and a third quantum dot material may be a blue quantum dot light-emitting material.

Since a difference between a highest occupied molecular orbital (HOMO) energy level of the red quantum dot light-emitting material and a HOMO energy level of a carrier transport material, a difference between a HOMO energy level of the green quantum dot light-emitting material and the HOMO energy level of the carrier transport material, a difference between a HOMO energy level of the blue quantum dot light-emitting material and the HOMO energy level of the carrier transport material are increasingly large, the hole injection ability shows that the first light-emitting device 201 is stronger than the second light-emitting device 202, and the second light-emitting device 202 is stronger than the third light-emitting device 203 (the first light-emitting device 201 > the second light-emitting device 202 > the third light-emitting device 203), and the electron injection ability shows that the first light-emitting device 201 is the same as the second light-emitting device 202, and the second light-emitting device 202 is stronger than the third light-emitting device 203 (the first light-emitting device 201 = the second light-emitting device 202 > the third light-emitting device 203). The electron injection ability of the first light-emitting device 201 is greater than the hole injection ability of the first light-emitting device 201, and therefore, at least one auxiliary layer 71 on a side of the first light-emitting device 201 away from the base 11 can effectively block the injection of electrons, which can make the transport of carriers (including electrons and holes) of the first light-emitting device 201 more balanced, which is conducive to improving the external quantum efficiency of the light-emitting device 20.

Based on the above specific implementations, the following experimental data is provided for a configuration in which an auxiliary layer 71 is provided on a side of a light-emitting pattern 23a contained in the first light-emitting device 201 away from the base 11 after the light-emitting pattern 23a contained in the first light-emitting device 201 is formed.

As shown in FIG. 13, the horizontal coordinate represents the wavelength of ultraviolet-visible light (in units of nm), and the vertical coordinate represents the absorbance unit (denoted as a.u.).

Here, "Third Blank Control" refers to a configuration in which no auxiliary layer 71 is provided on a side of the light-emitting pattern 23a contained in the first light-emitting device 201 away from the base 11; and "Fifth Structure" refers to a configuration in which an auxiliary layer 71 is provided on a side of the light-emitting pattern 23a contained in the first light-emitting device 201 far away from the base 11. There are two curves both indicating "Fifth Structure" which means that the two curves are test results by sampling a same sample at different regions.

As can be seen from FIG. 13, the curve of "Third Blank Control" has no absorption peak at 400 nm, while the two curves of "Fifth Structure" each have an absorption peak at 400 nm, where the absorption peak at 400 nm is an absorption peak of the material of the auxiliary layer 71. This indicates that the light-emitting pattern 23a contained in the first light-emitting device 201 is provided with the auxiliary layer 71 on its side away from the base 11.

In some embodiments, a material of the light-emitting pattern 23a contained in the first light-emitting device 201, as shown in FIG. 2, includes a first cross-linked quantum dot material, where the first cross-linked quantum dot material is generated by cross-linking a first quantum dot material and a second photosensitive material under light radiation.

In some embodiments, the first quantum dot material includes a quantum dot body and a first ligand material coordinated on the quantum dot body. The quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot. In the ABX₃ type perovskite quantum dot, A is one or more of CH₃NH₃⁺ (Methylamine), NH₂CH=NH₂ (Formamidine), and Cs⁺, B is one or two of Pb²⁺ and Sn²⁺, and X is one or more of Cl⁻, Br⁻, and I⁻. The ABX₃ type perovskite quantum dots include CH₃NH₃PbBr₃, CH₃NH₃PbCl₃, CH₃NH₃PbI₃, CsPbBr₃, CsPbCl₃, and CsPbl₃.

For example, the first ligand material coordinated on the quantum dot body is used to be cross-linked with the second photosensitive material. The first ligand material may contain a C-H bond, where the C-H bond can be cross-linked with the second photosensitive material under light radiation. The first ligand material may contain a double bond, where the double bond in the first ligand material can be cross-linked with the second photosensitive material under light radiation. These are described with specific reference to the subsequent contents and are not described in detail here.

For example, the Group IIB-VIA quantum dot is selected from one or more of: a binary compound such as CdSe, CdTe, ZnS, ZnSe, ZnTe, ZnO, HgS, HgSe, HgTe, MgSe, or MgS, a ternary compound such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, or a mixture thereof, and a quaternary compound such as HgZnTeS, CdZnSeS, CdZnSeTe, CdZnSTe, CdHgSeS, CdHgSeTe, CdHgSTe, HgZnSeS, HgZnSeTe, HgZnSTe, or a mixture thereof, which are not limited thereto.

The Group IIIA-VA quantum dot is selected from: a binary compound such as GaN, GaP, GaAs, GaSb, AIN, AlP, AlAs, AlSb, InN, InP, InAs, InSb, or a mixture thereof, a ternary compound such as GaNP, GaNAs, GaNSb, GaPAs, GaPSb, AINP, AlNAs, AlNSb, AlPAs, AlPSb, InNP, InNAs, InNSb, InPAs, InPSb, or a mixture thereof, and a quaternary compound such as GaAlNP, GaAINAs, GaAINSb, GaAlPAs, GaAlPSb, GaInNP, GaInNAs, GaInNSb, GaInPAs, GaInPSb, InAlNP, InAINAs, InAINSb, InAlPAs, InAlPSb, or a mixture thereof, which are not limited thereto.

The Group IVA-VIA quantum dot is selected from: a binary compound such as SnS, SnSe, SnTe, PbS, PbSe, PbTe, or a mixture thereof, a ternary compound such as SnSeS, SnSeTe, SnSTe, PbSeS, PbSeTe, PbSTe, SnPbS, SnPbSe, SnPbTe, or a mixture thereof, and a quaternary compound such as SnPbSSe, SnPbSeTe, SnPbSTe, or a mixture thereof, which are not limited thereto. The Group IVA-VIA quantum dot is, for example, selected from: a simple (mono) semiconductor such as Si, Ge, or a mixture thereof, and a binary semiconductor compound such as SiC, SiGe, or a mixture thereof, which are not limited thereto.

The quantum dot with core-shell structure means that one of the materials is a material of the core and the other is a material of the shell. For example, a quantum dot of CdS/ZnS means that a material of the core of the quantum dot is CdS and a material of the shell is ZnS.

In some other embodiments, the quantum dot body may be any of other nanoscale materials, such as a nanorod, a nanosheet, or the like. Components of other nanoscale materials may include at least one of CdS, CdSe, CdTe, ZnSe, InP, PbS, CuInS₂, ZnO, CsPbCl₃, CsPbBr₃, CsPhI₃, CdS/ZnS, CdSe/ZnS, ZnSe, InP/ZnS, PbS/ZnS, InAs, InGaAs, InGaN, GaNk, ZnTe, Si, Ge, and C.

For example, the quantum dot body may include a cadmium (Cd)-free quantum dot. The cadmium-free quantum dot is a quantum dot that does not contain cadmium (Cd). Cadmium (Cd) can cause serious environmental/health issues, so non-Cd-based quantum dots can be effectively used.

In some embodiments, the first ligand material is selected from any one of an organic acid, an organic amine, an organophosphorus, and an organic thiol.

For example, in a case where the first ligand material contains a double bond and the double bond is located at the end of its carbon chain, the first ligand material may be represented by the following formula: 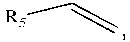 where R₅ is selected from any one of a C1 to C40 carbon chain with a carboxyl group, a C1 to C40 carbon chain with an amino group, and a C1 to C40 carbon chain with a sulfhydryl group; and any one of the carboxyl group, the amino group, and the sulfhydryl group is connected to the quantum dot body by coordination.

It will be noted that a "Cm" carbon chain herein refers to a carbon chain containing a total of m carbon (C) atoms.

Here, the first ligand material contains a C-H bond. In a case where the first ligand material is cross-linked with the second photosensitive material by a C-H insertion reaction, the first ligand material may be represented by the following formula for convenience of representation: 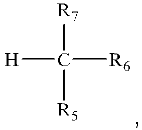 where R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40.

R₅, as shown above, is selected from any one of a C1 to C40 carbon chain with a carboxyl group, a C1 to C40 carbon chain with an amino group, and a C1 to C40 carbon chain with a sulfhydryl group; and any one of the carboxyl group, the amino group, and the sulfhydryl group is connected to the quantum dot body by coordination.

Two formulas are used to represent the structure of the first ligand material because of the reasons below.

Under light radiation, the C-H bond in the first ligand material can be cross-linked with the second photosensitive material. The specific cross-linking reaction is described in the subsequent contents and will not be described in detail here.

In a case where a double bond exists in the structure of the first ligand material, the double bond in the first ligand material can be cross-linked with the second photosensitive material under light radiation. The specific cross-linking reaction is described in the subsequent contents and will not be described in detail here.

In some embodiments, the second photosensitive material includes a second photosensitive group, the second photosensitive group undergoing a cross-linking reaction with a quantum dot material under light radiation. The quantum dot material includes the first quantum dot material.

In some embodiments, the second photosensitive material includes a benzophenone group, the benzophenone group being the second photosensitive group.

In some examples, the second photosensitive material includes: ethylene glycol di(3-benzoylbenzoate), propylene glycol di(3-benzoylbenzoate), 1,4-butanediol bis(3-benzoylbenzoate), 1,2-ethanedithiol bis(3-benzoylbenzoate), 1,3-propanedithiol bis(3-benzoylbenzoate), 1,4-butanedithiol bis(3-benzoylbenzoate), propylene glycol tri(3-benzoylbenzoate) di(3-benzoylbenzoate), 1,2,3-propanetrithiol tris(3-benzoylbenzoate), pentaerythritol tetra(3-benzoylbenzoate), pentaerythrityl tetra(3-benzoylbenzoate), di(pentaerythritol) hex(3-benzoylbenzoate), or di(pentaerythrityl tetrathio) hex(3-benzoylbenzoate).

It will be noted that hydrogen atoms on the benzene ring of the first photosensitive material can be replaced by other functional groups, such as methoxy, ethoxy, propoxy, tetrahydrofuranyl, methylamino, ethylamino, propylamino, pyrrolidinyl, halogen group, methylthio, ethylthio, propylthio, and tetrahydrothiophenyl, which are not limited here.

The second photosensitive material containing a benzophenone group can be cross-linked with the first ligand material coordinated on the quantum dot body. For example, benzophenone is photolyzed to form a triplet ketone intermediate under light radiation (hv), with a structure shown in the following formula, in which the C^{•}-O^{•} in the triplet ketone intermediate will attack a double bond or C-H bond nearby to undergo a cross-linking reaction.

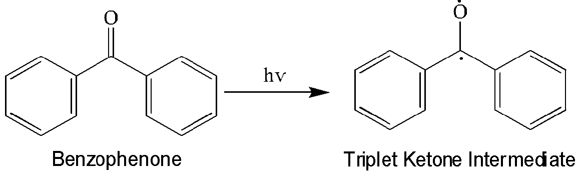

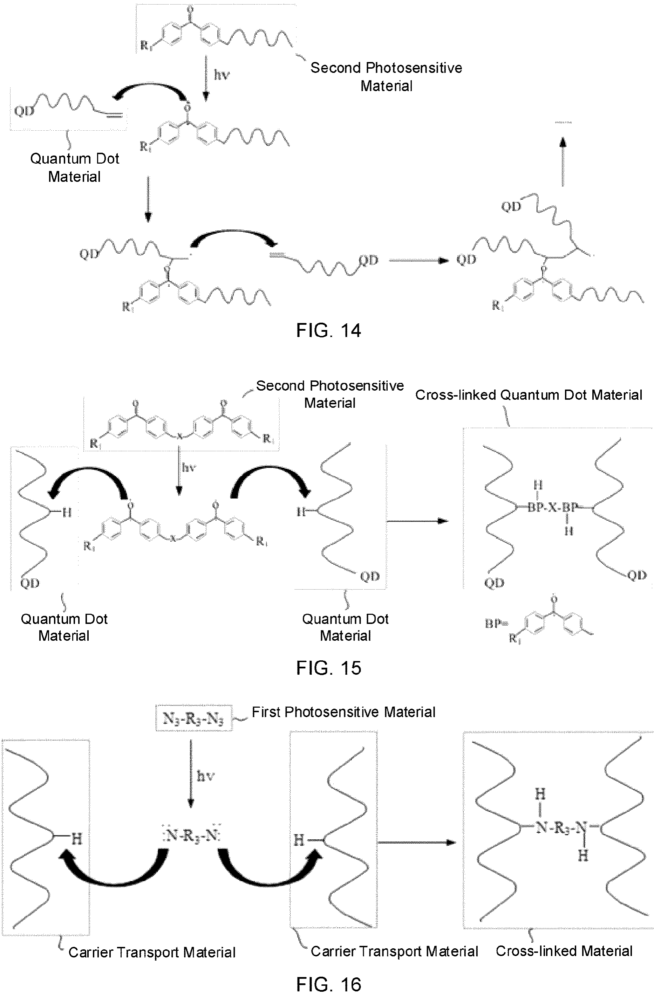

In some examples, as shown in FIG. 14, the C^{•}-O^{•} contained in the triplet ketone intermediate in the second photosensitive material attacks a double bond nearby, that is, the C^{•}-O^{•} contained in the triplet ketone intermediate in the second photosensitive material attacks the double bond in the first ligand material coordinated on the quantum dot body (QD). The oxygen free radical (O^{•}) in the C^{•}-O^{•} contained in the triplet ketone intermediate will attack the double bond in the first ligand material, enabling the double bond in the first ligand material to be opened.

After the double bond in the first ligand material is opened, two carbon free radicals (C^{•}) are formed, in which one carbon free radical (C^{•}) combines with the oxygen free radical (O^{•}) in the second photosensitive material, and the other carbon free radical (C^{•}) will further attack a next double bond in the first ligand material. After this double bond is opened, carbon free radicals (C^{•}) are formed, and one carbon free radical (C^{•}) further attacks a next double bond in the first ligand material, thereby enabling the second photosensitive material and the quantum dot material (including the first quantum dot material) to be cross-linked to form a cross-linked quantum dot material (including the first cross-linked quantum dot material).

Here, when the carbon radical (C^{•}) attacks the C-H bond in the first ligand material, the cross-linking reaction stops.

For example, the first cross-linked quantum dot material includes any one of the structures shown in the following general formula IV: 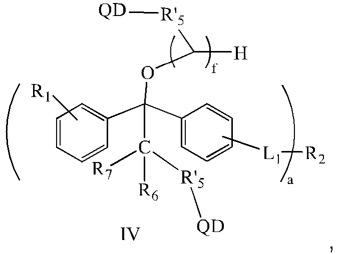 where L₁ is selected from any one of a single bond, an ester bond, an ether bond, and a thioether bond; R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; and R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40.

The QD represents any one quantum dot body. R'₅ is selected from a remaining structure of R₅ with one hydrogen removed from any one of a carboxyl group, an amino group, and a sulfhydryl group therein. R₅ is selected from any one of a C1 to C40 carbon chain with a carboxyl group, a C1 to C40 carbon chain with an amino group, and a C1 to C40 carbon chain with a sulfhydryl group; and any one of the carboxyl group, the amino group, and the sulfhydryl group is connected to the quantum dot body by coordination. a is a positive integer taking a value greater than or equal to 1, and f is a positive integer taking a value greater than or equal to 1.

Here, the value of f means that in the cross-linking reaction, f molecules of the first ligand material containing double bonds participate in the cross-linking reaction. When the carbon free radical (C^{•}) attacks the C-H bond in the first ligand material, the cross-linking reaction stops.

In some embodiments, as shown in FIG. 15, the C^{•}-O^{•} contained in the triplet ketone intermediate in the second photosensitive material attacks the nearby C-H bond, that is, the C^{•}-O^{•} contained in the triplet ketone intermediate in the second photosensitive material attacks the C-H bond in the first ligand material coordinated on the quantum dot body (QD). The C^{•}-O^{•} contained in the triplet ketone intermediate will attack the C-H bond in the first ligand material, enabling the C-H bond in the first ligand material to be opened.

After the C-H bond in the first ligand material is opened, a carbon free radical (C^{•}) and a hydrogen free radical (H^{•}) are formed. The oxygen free radical (O^{•}) in the second photosensitive material will attack the hydrogen free radical (H^{•}) to form a hydrogen-oxygen bond (-OH), and the carbon free radical (C^{•}) in the second photosensitive material will attack the carbon free radical (C^{•}) in the first ligand material to form a carbon-carbon bond, so that the second photosensitive material and the quantum dot material (including the first quantum dot material) are cross-linked to form a cross-linked quantum dot material (including the first cross-linked quantum dot material).

Here, "X" shown in FIG. 15 represents a connecting group connecting two benzophenone groups.

For example, the first cross-linked quantum dot material includes any one of the structures shown in the following general formula V: 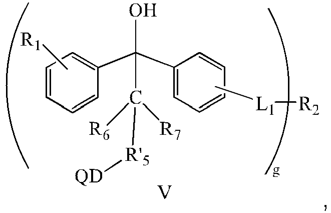 where L₁ is selected from any one of a single bond, an ester bond, an ether bond, and a thioether bond. R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40.

The QD represents any one quantum dot body. R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination.

g is a positive integer taking a value greater than or equal to 2.

For example, g takes a value of 2, 3, 4, 5 or 6, which is not limited here. By setting g being a positive integer taking a value greater than or equal to 2, each benzophenone group in the second photosensitive material can be connected to one molecule of the first ligand material. Therefore, molecules of the second photosensitive material containing g benzophenone groups can achieve the connection with g molecules of the quantum dot material (including the first quantum dot material) to form the cross-linked quantum dot material (including the first cross-linked quantum dot material).

In some embodiments, as shown in FIG. 3, the material of the hole transport layer 41 includes any one of materials containing at least one of a carbazole group, a carbazole derivative group, a triphenylamine group, and a triphenylamine derivative group. Alternatively, the material of the electron transport layer 43 includes: nano zinc oxide (ZnO) or metal-doped nano zinc oxide, and a second ligand material coordinated on a surface of the nano zinc oxide or metal-doped nano zinc oxide.

For example, the material of the hole transport layer 41 includes: poly[(9,9-dioctylfluorenyl-2,7-diyl)-alt-(4,4'-(N-(4-butylphenyl))-diphenylamine)] (TFB), poly(N-vinyl carbazole) (PVK), or poly[bis(4-phenyl)(4-butylphenyl)amine] (Poly-TPD).

For example, the material of the electron transport layer 43 includes: nanoparticle zinc oxide (ZnO) or sol-gel zinc oxide (ZnO), and a second ligand material coordinated on the nanoparticle zinc oxide (ZnO) or sol-gel zinc oxide (ZnO).

For example, the metal-doped nano zinc oxide includes zinc oxide (ZnO) doped with magnesium (Mg), aluminum (Al), zirconium (Zr), or yttrium (Y). The second ligand material includes, for example, an organic material such as acetamide.

The material of the first carrier transport layer 50 is represented as a first carrier transport material. The first carrier transport material contains a C-H bond. A carrier transport material containing a C-H bond is represented by the following formula:

H-C-(Y),

where Y is a remaining structure of the first carrier transport material with the C-H bond (also referred to as a C-H insertion group) removed.

For example, the carrier transport material is TFB, TFB standing for poly[(9,9-dioctylfluorenyl-2,7-diyl)-alt-(4,4'-(N-(4-butylphenyl))-diphenylamine)], then TFB can be represented by the following formula:

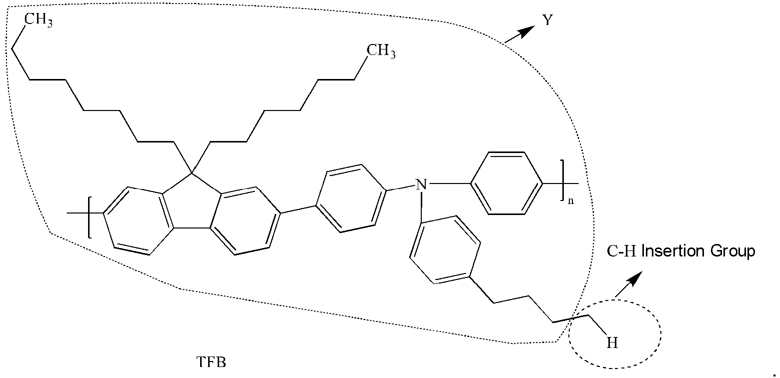

It will be noted that the C-H bond contained in the first carrier transport material will undergo a C-H insertion reaction with the first photosensitive material to form the cross-linked material (including the first cross-linked material).

In some embodiments, the first photosensitive material includes a first photosensitive group, and the first photosensitive group undergoes a cross-linking reaction with a carrier transport material under light radiation; and the carrier transport material includes the first carrier transport material. Furthermore, the first photosensitive group and the second photosensitive group are different.

For example, the first photosensitive material includes an azide group, that is, the azide group is the first photosensitive group.

Under light radiation, the first photosensitive material containing the azide group (-N₃) undergoes a cross-linking reaction with the C-H bond in the carrier transport material (including the first carrier transport material).

In some embodiments, the first cross-linked material is selected from any one of the structures shown in the following general formula VI: 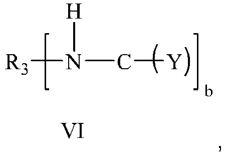 where R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. b is a positive integer taking a value greater than or equal to 2. Y represents the remaining structure of the first carrier transport material with the C-H bond removed, and the C-H bond is used for undergoing a cross-linking reaction with the first photosensitive group of the first photosensitive material under light radiation. An exemplary description of Y can refer to the foregoing and will not be repeated here.

For example, as shown in FIG. 16, b takes a value of 2, that is, the first photosensitive material contains two azide groups (-N₃). Each azide group (-N₃) attacks one molecule of the carrier transport material, and through the C-H insertion reaction, the first photosensitive material and the carrier transport material are cross-linked to form a cross-linked material (including the first cross-linked material). The first cross-linked material has a low solubility in a solvent, that is, the first cross-linked material is not soluble in the developing solution during development.

For example, first photosensitive materials containing azide groups include: 1,2-diazidoethane (with the molecular formula of C₂H₄N₆), 1,2-diazidopropane, 1,2-diazidobutane, 1,11-diazido-3,6,9-trioxaundecane (with the molecular formula of C₈H₁₆N₆O₃), ethylene glycol di(4-azidobenzoate), di(4-azido-2,3,5,6-tetrafluorobenzoate) (with the molecular formula of C₁₆H₄F₈N₆O₄), (1,3,4-triazido)isobutane, (1,3,5-triazido)isopentane, trithioglycerin tris(4-azido)benzoate, (1,3,4,5-tetraazido)neopentane, pentaerythritol tetra(4-azido benzoate), pentaerythrityl tetra (4-azidobenzoate), di(pentaerythritol) hex(4-azidobenzoate), and di(pentaerythrityl tetrathio) hex(4-azidobenzoate), but are not limited here.

For example, the first photosensitive material includes a diazirine group, that is, the diazirine group is the first photosensitive group.

Under light radiation, the first photosensitive material containing the diazirine group undergoes a cross-linking reaction with the C-H bond in the carrier transport material (including the first carrier transport material).

In some embodiments, the first cross-linked material is selected from any one of the structures shown in the following general formula XI-A: 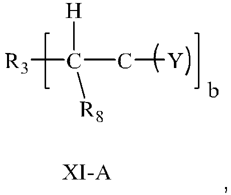 where R₈ and R₃ are each independently selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. b is a positive integer taking a value greater than or equal to 2. Y represents the remaining structure of the first carrier transport material with the C-H bond removed, and the C-H bond is used for undergoing a cross-linking reaction with the first photosensitive group of the first photosensitive material under light radiation. An exemplary description of Y can refer to the foregoing and will not be repeated here.

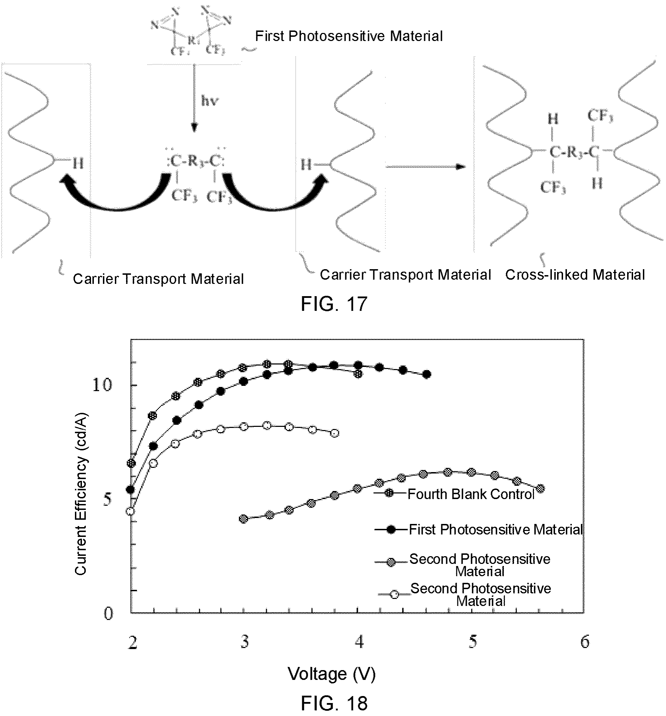

For example, as shown in FIG. 17, b takes a value of 2, that is, the first photosensitive material contains two diazirine groups. Each diazirine group attacks one molecule of the carrier transport material, and through the C-H insertion reaction, the first photosensitive material and the carrier transport material are cross-linked to form a cross-linked material (including the first cross-linked material). The first cross-linked material has a low solubility in a solvent, that is, the first cross-linked material is not soluble in the developing solution during development.

For example, first photosensitive materials containing diazirine groups include: 1,2-bis-diazirine ethane, 1,2-bis-diazirine propane, 1,2-bis-diazirine butane, 1,11-bis-diazirine-3,6,9-trioxaundecane, ethylene glycol di(4-diazirinebenzoate), ethylene glycol di(4-azidobenzoate) di(4-diazirine-2,3,5,6-tetrafluorobenzoate), (1,3,4-tri-diazirine)isobutane, (1,3,5-tri-diazirine)isopentane, (1,3,4,5-tetra-diazirine)neopentane, pentaerythritol tetra(4-diazrine benzoate), pentaerythrityl tetra (4-diazrine benzoate), di(pentaerythritol) hex(4-diazrine benzoate), and di(pentaerythrityl tetrathio) hex(4-diazrine benzoate), but are not limited here.

For example, the first photosensitive material includes a diazo group, that is, the diazo group is the first photosensitive group.

Under light radiation, the first photosensitive material containing the diazo group undergoes a cross-linking reaction with the C-H bond in the carrier transport material (including the first carrier transport material).

For example, first photosensitive materials containing diazo groups include: 1,2-bis-diazo ethane, 1,2-bis-diazo propane, 1,2-bis-diazo butane, 1,11-bis-diazo-3,6,9-trioxaundecane, ethylene glycol di(4-diazobenzoate), ethylene glycol di(4-azidobenzoate) di(4-diazo-2,3,5,6-tetrafluorobenzoate), (1,3,4-tri-diazo)isobutane, (1,3,5-tri-diazo)isopentane, (1,3,4,5-tetra-diazo)neopentane, pentaerythritol tetra(4-diazo benzoate), pentaerythrityl tetra (4-diazobenzoate), di(pentaerythritol) hex(4-diazobenzoate), and di(pentaerythrityl tetrathio) hex(4-diazobenzoate), but are not limited here.

In some embodiments, the first cross-linked material is selected from any one of the structures shown in the following general formula VII: 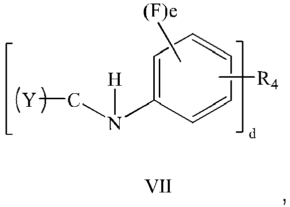 where R₄ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. d is a positive integer taking a value greater than or equal to 2. e takes any one of values 0, 1, 2, 3 and 4. Y represents the remaining structure of the first carrier transport material with the C-H bond removed, and the C-H bond is used for undergoing a cross-linking reaction with the first photosensitive group of the first photosensitive material under light radiation. An exemplary description of Y can refer to the foregoing and will not be repeated here.

In some embodiments, the first cross-linked quantum dot material is generated by cross-linking the first quantum dot material, the first photosensitive material, and the second photosensitive material under light radiation.

Descriptions of the second photosensitive material and the first photosensitive material can refer to the foregoing and will not be repeated here.

That is, there are two types of photosensitive materials in the first cross-linked quantum dot material, namely, the first photosensitive material and the second photosensitive material.

The material description of the first quantum dot material can refer to the foregoing and will not be repeated here.

For example, under light radiation, the first photosensitive material containing the azide group (-N₃) undergoes a cross-linking reaction with the C-H bond in the first ligand material.

In some embodiments, the first cross-linked quantum dot material includes any one of the structures shown in the following general formula VIII: 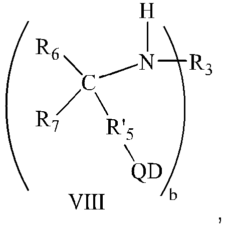 where QD represents any one quantum dot body. R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination. R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40. b is a positive integer taking a value greater than or equal to 2.

By setting two types of photosensitive materials and the quantum dot material (including the first quantum dot material) to be cross-linked to generate the cross-linked quantum dot material (including the first cross-linked quantum dot material), since the speed of the C-H insertion cross-linking reaction of the first photosensitive material containing the azide group (-N₃) is greater than the speed of the C-H insertion cross-linking reaction of the second photosensitive material containing the benzophenone group, the addition of the first photosensitive material can increase the speed of the cross-linking reaction.

In some embodiments, the light-emitting pattern 23, as shown in FIG. 3, contains a cross-linked quantum dot material, and the first carrier transport layer 50 in contact with the light-emitting pattern 23 containing the cross-linked quantum dot material contains a cross-linked material, then the light-emitting pattern 23 and the first carrier transport layer 50 containing the cross-linked material are connected with a first cross-linked connecting material therebetween.

For example, in a case where the C-H bond in the first ligand material of the quantum dot material undergoes a cross-linking reaction through a C-H insertion reaction, the quantum dot material can be represented by the following formula:

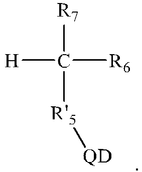

A C-H bond of one molecule in the carrier transport material and a C-H bond of one molecule in the first ligand material of the quantum dot material undergo a carbon-hydrogen insertion reaction with a benzophenone group of the same molecule in the second photosensitive material under light radiation to form a cross-linked connecting material connected between the carrier transport layer and the light-emitting pattern 23.

For example, in a case where the quantum dot material undergoes a cross-linking reaction through a double bond addition reaction of the first ligand material, the quantum dot material can be represented by the following formula:

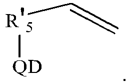

The first ligand material contains a double bond. The double bond in the first ligand material of the quantum dot material undergoes an addition reaction with the benzophenone group in the second photosensitive material. This addition reaction is terminated by the participation of the C-H bond in the carrier transport material to form a cross-linked connecting material connected between the carrier transport layer and the light-emitting pattern 23.

The first cross-linked connecting material may enable the first carrier transport layer 50 in contact therewith to be more firmly bonded to the light-emitting pattern 23.

Based on the above specific embodiments, in order to objectively evaluate the technical effect of the technical solution provided in the present disclosure, hereinafter, the technical effect of the technical solution provided in the present disclosure will be verified by verification experiments.

By using the second photosensitive material as the photosensitive material for forming the cross-linked quantum dot material (including the first cross-linked quantum dot material), it may be ensured that the light-emitting pattern 23 has a high film retention rate, and it will not cause a decrease in the current efficiency of the light-emitting device 20.

For example, as shown in FIG. 6, the first light-emitting device 201 includes a first electrode 21, a hole injection layer 41, a hole transport layer 131, a light-emitting pattern 23a, an auxiliary layer 71, an electron transport layer 43, and a second electrode 22, which are arranged in a stack on the base 11 in sequence.

A description of the structure of the first light-emitting device 201 can refer to the foregoing and will not be repeated herein.

Here, the hole transport layer 131 contains a first cross-linked material generated by cross-linking a carrier transport material and a first photosensitive material under light radiation.

For example, as shown in FIG. 18, the horizontal coordinate in the graph represents the voltage (in units of V), and the vertical coordinate represents the current efficiency (in units of cd/A) of the first light-emitting device 201. This example provides curves of the current efficiency with the voltage of four types of first light-emitting devices 201, in which the four types of first light-emitting devices 201 in this example each contain a different material in its light-emitting pattern 23a, and have the same thickness and same materials of the remaining film layers.

Here, "Fourth Blank Control" means that there is no cross-linked quantum dot material formed by the second photosensitive material and the first photosensitive material in a light-emitting pattern 23a; "Second Photosensitive Material" means that a material of a light-emitting pattern 23a is a cross-linked quantum dot material formed by the second photosensitive material and the quantum dot material; and "First Photosensitive Material" means that a material of a light-emitting pattern 23a is a cross-linked quantum dot material formed by the first photosensitive material and the quantum dot material; where the two types of "First Photosensitive Material" indicate that the first photosensitive materials used are different.

It can be seen from FIG. 18 that adding "First Photosensitive Material" will cause a decrease in the current efficiency of the light-emitting device 20, while the "Second Photosensitive Material" will not cause a decrease in the current efficiency of the light-emitting device 20.

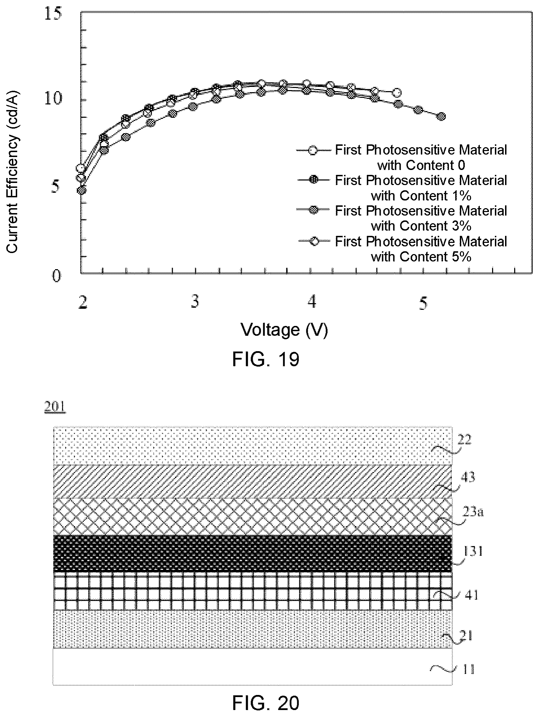

Moreover, as shown in FIG. 19, this example provides curves of the current efficiency with the voltage of four types of first light-emitting devices 201, in which the four types of first light-emitting devices 20 in this example each contain a different content of the second photosensitive material in its light-emitting pattern 23a, and have the same thickness and same materials of the remaining film layers.

Here, "Second Photosensitive Material with Content 0" means that the second photosensitive material is not added when forming the light-emitting pattern 23a; "Second Photosensitive Material with Content 1%" means that a ratio of the quality of the second photosensitive material to the quality of the first quantum dot material is 1%; "Second Photosensitive Material with Content 3%" means that a ratio of the quality of the second photosensitive material to the quality of the first quantum dot material is 3%; "Second Photosensitive Material with Content 5%" means that a ratio of the quality of the second photosensitive material to the quality of the first quantum dot material is 5%.

It can be seen from FIG. 19 that as the content of the second photosensitive material increases, the current efficiency of the light-emitting device 20 does not change significantly. Therefore, it can be further verified that the second photosensitive material will not damage the performance of the light-emitting device 20.

Adding the first photosensitive material into the hole transport layer 131 will not affect the performance of the light-emitting device 20.

For example, as shown in FIG. 20, the first light-emitting device 201 includes a first electrode 21, a hole injection layer 41, a hole transport layer 131, a light-emitting pattern 23a, an electron transport layer 43, and a second electrode 22, which are arranged in a stack on the base 11 in sequence.

A description of the structure of the first light-emitting device 201 can refer to the foregoing and will not be repeated herein.

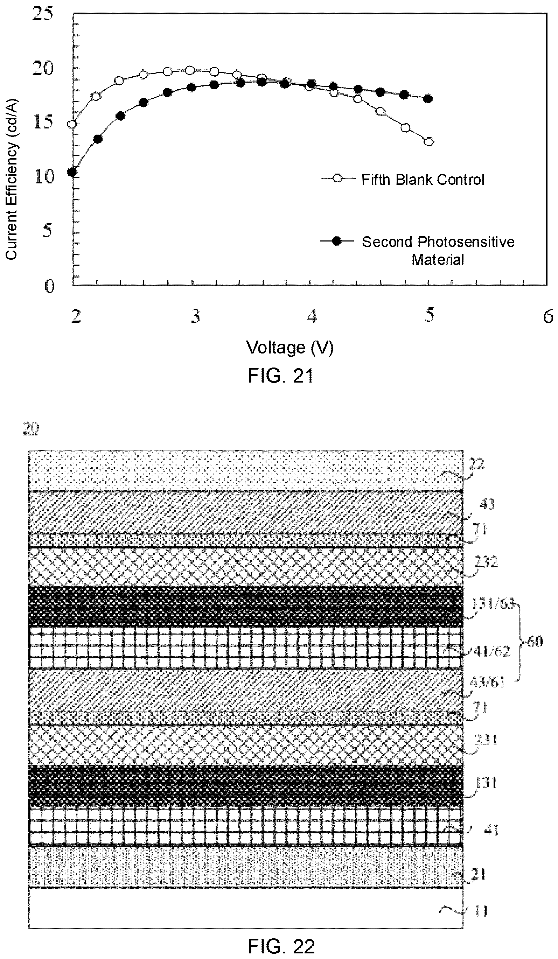

For example, as shown in FIG. 21, the horizontal coordinate in the graph represents the voltage (in units of V), and the vertical coordinate represents the current efficiency (in units of cd/A) of the light-emitting device 20. This example provides curves of the current efficiency with the voltage of two types of first light-emitting devices 201, in which the two types of first light-emitting devices 201 in this example each contain a different material in its hole transport layer 131, and have the same thickness and same materials of the remaining film layers.

Here, "Fifth Blank Control" refers to a hole transport layer 131 without adding photosensitive material; and "First Photosensitive Material" refers to a material of a hole transport layer 131 including the first cross-linked material formed by the first carrier transport material and the first photosensitive material, for example, a ratio of the quality of the first photosensitive material to the quality of the first carrier transport material is 15%.

It can be seen from FIG. 21 that after adding the first photosensitive material into the first light-emitting device 201, the current efficiency of the light-emitting device 20 is not greatly affected. Therefore, adding the first photosensitive material into the first light-emitting device 201 will not affect the performance of the light-emitting device 20.

It will be noted that a material of each film layer, as well as a material existing between film layers, can be characterized by nuclear magnetic resonance, infrared, mass spectrometry, and XPS analysis (X-ray photoelectron spectroscopy).

For example, it is possible to determine the distribution of elements in each section by scanning sections of the device with a TEM-EDS (transmission electron microscopy-energy dispersive spectroscopy) and performing an elemental analysis, so as to determine whether the second photosensitive material containing a benzophenone group or the first photosensitive material containing an azide group (-N₃) has been used. For example, if the first photosensitive material containing an azide group (-N₃) is added to the TFB, elemental analysis will show the presence of the element nitrogen (N) as well as a secondary amine group.

As shown in FIG. 22, the light-emitting device 20 includes multiple light-emitting patterns 23 arranged in a stack, and a charge generation layer 60 is provided between every two adjacent light-emitting patterns 23 in the multiple light-emitting patterns 23.

For example, the light-emitting device 20 includes two light-emitting patterns 23. For example, the light-emitting device 20 includes a first electrode 21, a hole injection layer 41, a hole transport layer 131, a first light-emitting pattern 231, a charge generation layer 60, a second light-emitting pattern 232, an electron transport layer 43, and a second electrode 22, which are arranged in a stack in sequence.

For example, the charge generation layer 60 includes a third carrier transport layer 61, a second carrier injection layer 62, and a fourth carrier transport layer 63, in which a material of the third carrier transport layer 61 is the same as the material of the electron transport layer 43, a material of the second carrier injection layer 62 is the same as the material of the hole injection layer 41, and a material of the fourth carrier transport layer 63 is the same as the material of the hole transport layer 131.

That is, the light-emitting device 20 is a stacked light-emitting device, which is characterized by long life and high efficiency.

For example, as shown in FIG. 22, at least one auxiliary layer 71 may be provided between the light-emitting pattern 23 and the electron transport layer 43, and the provision of the auxiliary layer 71 may enable a more balanced transport of carriers (including electrons and holes) of the light-emitting device 20, which is conducive to improving the external quantum efficiency of the light-emitting device 20.

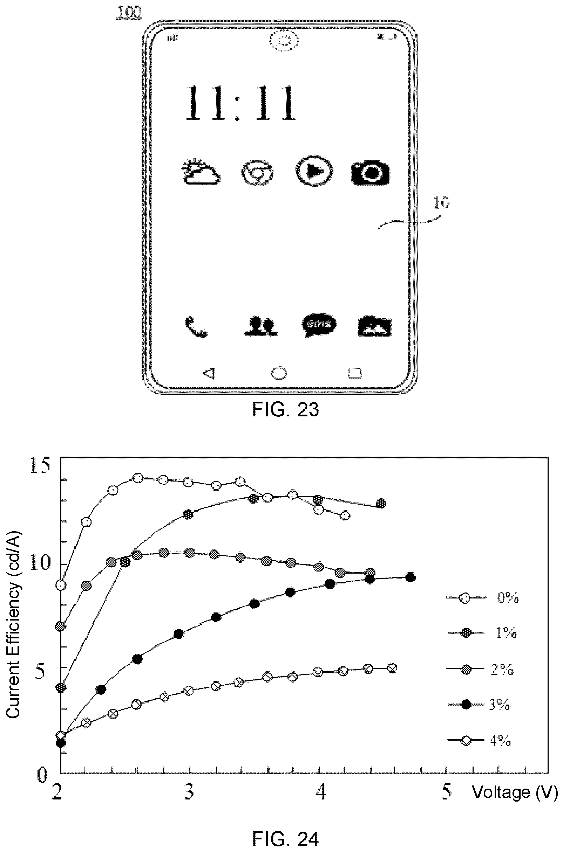

As shown in FIG. 23, some embodiments of the present disclosure provide a light-emitting apparatus 100, which includes the light-emitting substrate 10 as described in any of the above embodiments.

Of course, the light-emitting apparatus 100 may further include other components, for example, it may include a driving circuit for providing electrical signals to the light-emitting substrate 10 to drive the light-emitting substrate 10 to emit light, where this circuit may be referred to as a control circuit. The light-emitting apparatus 100 may further include a circuit board and/or an integrated circuit (IC) electrically connected to the light-emitting substrate 10.

In some embodiments, the light-emitting apparatus 100 may be a lighting apparatus, and in this case, the light-emitting apparatus 100 serves as a light source to realize a lighting function. For example, the light-emitting apparatus 100 may be a backlight module in a liquid crystal display device, a lamp used for internal or external illumination, or a variety of signal lamps.

In some other embodiments, the light-emitting apparatus 100 may be a display apparatus, and in this case, the light-emitting substrate 10 is a display substrate for realizing functions of displaying images (i.e., pictures). The light-emitting apparatus 100 may include a display or a product containing a display. Here, the display may be a flat panel display (FPD), a micro-display, or the like. If classified according to whether the user can see the scene on the back of the display, the display may be a transparent display or an opaque display. If classified according to whether the display can be bent or curled, the display may be a flexible display or a normal display (which can be referred to as a rigid display). For example, products containing displays may include a computer monitor, a television, a billboard, a laser printer with a display function, a telephone, a cellular phone, a personal digital assistant (PDA), a laptop computer, a digital camera, a portable camcorder, a viewfinder, a vehicle, a large wall area, a screen in a theater or stadium sign, and the like. The light-emitting apparatus 100 is illustrated in FIG. 23 as an example of a cell phone.

In some embodiments, a ratio of the quality of the first photosensitive material to the quality of the quantum dot material ranges from 0% to 1%, so that it can be ensured that the first photosensitive material containing an azide group (-N₃) does not affect the light-emitting efficiency of the light-emitting device 20, and the effect on the performance of the light-emitting device 20 can be negligible.

As shown in FIG. 24, the horizontal coordinate represents the voltage (in units of V), and the vertical coordinate represents the current efficiency (in units of cd/A) of the light-emitting device 20. In the figure, 0%, 1%, 2%, 3%, and 4% represent that the ratio of the quality of the first photosensitive material to the quality of the first quantum dot material is 0%, 1%, 2%, 3%, and 4%, respectively. As can be seen from the figure, the current efficiency of the light-emitting device 20 decreases significantly in a case where the ratio of the quality of the first photosensitive material to the quality of the first quantum dot material is greater than 1% (e.g., 2%, 3%, and 4%), and therefore, the range of the ratio of the quality of the first photosensitive material to the quality of the quantum dot material (any one of the first quantum dot material, the second quantum dot material, and the third quantum dot material) is set to be from 0% to 1%.

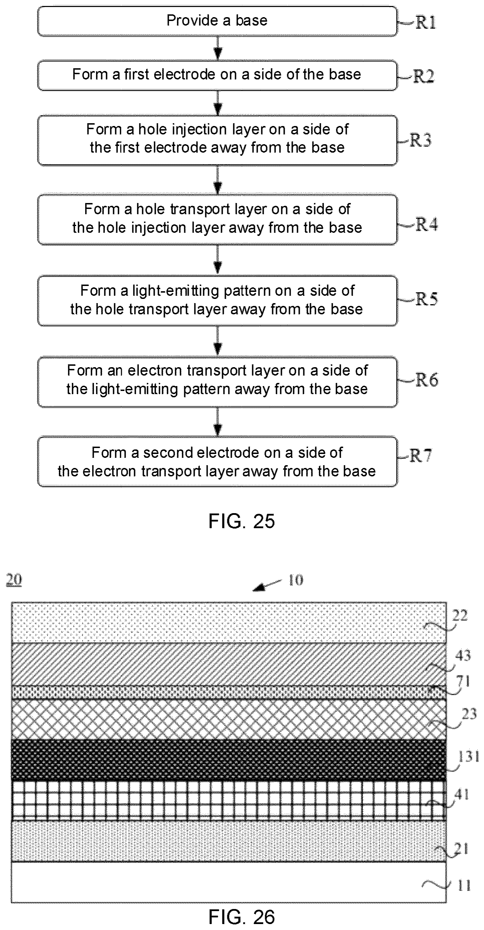

As shown in FIG. 25 and FIG. 26, in which FIG. 26 illustrates a film layer configuration of the light-emitting device 20 and FIG. 25 illustrates manufacturing steps of the light-emitting substrate, which include steps R1 to R7.

In R1, a base 11 is provided.

For example, the base 11 may be made of: an inorganic material, an organic material, a silicon wafer, or a composite layer. Exemplary inorganic materials may be glass, metal, etc.; and exemplary organic materials may be polycarbonate, polymethylmethacrylate, polyethylene terephthalate, polyethylene naphthalate two formic acid glycol ester, polyamide, polyethersulfone, or combinations thereof.

In R2, a first electrode 21 is formed on a side of the base 11.

For example, a glass substrate having a transparent electrode may be used, i.e., the transparent electrode is the first electrode 21. For example, the glass substrate having the transparent electrode is successively sonicated with deionized water and isopropyl alcohol for 15 minutes each, blown dry quickly with a nitrogen gun, then baked for 5 minutes at 150°C, and then treated with UV-ozone for half an hour in order to clean the surface of the transparent electrode and to increase the work function of the transparent electrode.

In R3, a hole injection layer 41 is formed on a side of the first electrode 21 away from the base 11.

For example, a hole injection material is spin-coated at a rotational speed of 3,000 rpm, and annealed in air at 130 °C for 20 minutes after completing spin-coating to dry the un-volatilized solvent.

For example, a hole injection material and a first photosensitive material are spin-coated at a rotational speed of 3,000 rpm, exposed by an exposure machine after completing spin-coating, and then annealed in air at 130 °C for 20 minutes to dry the non-volatilized solvent to form the hole injection layer 41 having a cross-linked material. The hole injection layer 41 having the cross-linked material is not susceptible to dissolution in order to provide the hole injection layer 41 with good stability, which is conducive to improving the stability of the light-emitting device 20.

In R4, a hole transport layer 131 is formed on a side of the hole injection layer 41 away from the base 11.

For example, in a glove box, a carrier transport material is spin-coated on the side of the hole injection layer 41 away from the base 11 at a rotational speed of 2,500 rpm for 45 seconds, and annealed in a nitrogen glove box at 120 °C for 15 minutes after completing spin-coating.

For example, in a glove box, a carrier transport material and a first photosensitive material are spin-coated on the side of the hole injection layer 41 away from the base 11 at a rotational speed of 2,500 rpm for 45 seconds, exposed by an exposure machine with an exposure wavelength of 320 nm and a dose of 10 mJ/cm² after completing spin-coating, and then annealed in a nitrogen glove box at 120 °C for 15 minutes to form the hole transport layer 131 having a cross-linked material. The hole transport layer 131 having the cross-linked material is not susceptible to dissolution in order to provide the hole transport layer 131 with good stability, which is conducive to improving the stability of the light-emitting device 20.

In R5, a light-emitting pattern 23 is formed on a side of the hole transport layer 131 away from the base 11.

For example, a quantum dot material is spin-coated on the side of the hole transport layer 131 away from the base 11 at a rotational speed of 3,000 rpm for 45 seconds, and annealed in a glove box at 80 °C for 10 minutes after completing spin-coating.

For example, a quantum dot material and a second photosensitive material, e.g., the second photosensitive material is 5% by quality, are spin-coated on the side of the hole transport layer 131 away from the base 11 at a rotational speed of 3,000 rpm for 45 seconds, exposed by an exposure machine with an exposure wavelength of 365 nm and a dose of 200 mJ/cm² after completing spin-coating, and then annealed in a glove box at 80 °C for 10 minutes to form the light-emitting pattern 23 having a cross-linked material. The light-emitting pattern 23 having the cross-linked material is not susceptible to dissolution in order to provide the light-emitting pattern 23 with good stability, which is conducive to improving the stability of the light-emitting device 20.

In R6, an electron transport layer 43 is formed on a side of the light-emitting pattern 23 away from the base 11.

For example, at least one auxiliary layer 71 may be formed on the side of the light-emitting pattern 23 away from the base 11 before the electron transport layer 43 is formed on the side of the light-emitting pattern 23 away from the base 11. Then, the electron transport layer 43 is formed on the side of the auxiliary layer 71 away from the base 11. The provision of the at least one auxiliary layer 71 may enable a more balanced transport of carriers (including electrons and holes) of the light-emitting device 20, which is conducive to improving the external quantum efficiency of the light-emitting device 20.

For example, a second carrier transport material is spin-coated on the side of the light-emitting pattern 23 away from the base 11 at a rotational speed of 1,500 rpm for 45 seconds, and annealed in a glove box at 80 °C for 10 minutes after completing spin-coating.

For example, a second carrier transport material and a first photosensitive material are spin-coated on the side of the light-emitting pattern 23 away from the base 11 at a rotational speed of 1,500 rpm for 45 seconds, exposed by an exposure machine after completing spin-coating, and then annealed in a glove box at 80 °C for 10 minutes to form the electron transport layer 43 having a cross-linked material. The electron transport layer 43 having the cross-linked material is not susceptible to dissolution in order to provide the electron transport layer 43 with good stability, which is conducive to improving the stability of the light-emitting device 20.

In R7, a second electrode 22 is formed on a side of the electron transport layer 43 away from the base 11.

For example, aluminum with a thickness of 100 nm is evaporated as the second electrode 22 within a vacuum evaporation chamber.

The light-emitting substrate 10 including light-emitting devices 20 of different structures may be manufactured by the steps R1 to R7.

The foregoing description is only specific embodiments of the present disclosure, but the scope of protection of the present disclosure is not limited thereto. Any changes or replacements that a person skilled in the art could conceive of within the technical scope of the present disclosure shall be included in the protection scope of the present disclosure. Therefore, the protection scope of the present disclosure shall be subject to the protection scope of the claims.

## Claims

1. A light-emitting substrate, comprising:
a base; and
a plurality of light-emitting devices provided on the base, wherein each light-emitting device in the plurality of light-emitting devices includes a first electrode, a second electrode, and a light-emitting pattern provided between the first electrode and the second electrode, the first electrode being closer to the base than the second electrode;
wherein the plurality of light-emitting devices include at least one first light-emitting device, and the at least one first light-emitting device includes a first carrier transport layer; and
a material of at least one of a light-emitting pattern contained in the at least one first light-emitting device and the first carrier transport layer includes a cross-linked material.

2. The light-emitting substrate according to claim 1, wherein the first carrier transport layer includes an electron transport layer, a hole transport layer, and a hole injection layer.

3. The light-emitting substrate according to claim 2, wherein the electron transport layer and the light-emitting pattern contained in the at least one first light-emitting device are provided therebetween with an auxiliary layer, and a material of the auxiliary layer is the same as a material of one layer in the first carrier transport layer.

4. The light-emitting substrate according to claim 3, wherein the material of the auxiliary layer is the same as the material of the hole transport layer, and the material of the auxiliary layer is a first cross-linked material, the first cross-linked material being generated by cross-linking of a hole transport material and a first photosensitive material under light radiation.

5. The light-emitting substrate according to claim 4, wherein the first cross-linked material is selected from any one of structures shown in following general formula VI: 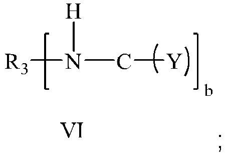
wherein R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
b is a positive integer taking a value greater than or equal to 2; and
Y represents a remaining structure of a first carrier transport material with a C-H bond removed, the C-H bond is used for undergoing a cross-linking reaction with a first photosensitive group of the first photosensitive material under light radiation.

6. The light-emitting substrate according to claim 4 or 5, wherein the first cross-linked material is selected from any one of structures shown in following general formula VII: 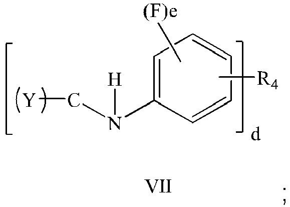
wherein R₄ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
d is a positive integer taking a value greater than or equal to 2;
e takes any one of values 0, 1, 2, 3 and 4; and
Y represents a remaining structure of a first carrier transport material with a C-H bond removed, the C-H bond is used for undergoing a cross-linking reaction with a first photosensitive group of the first photosensitive material.

7. The light-emitting substrate according to any one of claims 4 to 6, wherein the first cross-linked material is selected from any one of structures shown in following general formula XI-A: 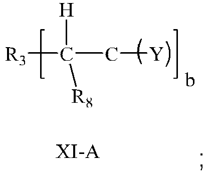
wherein R₈ and R₃ are each independently selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
Y represents a remaining structure of a first carrier transport material with a C-H bond removed, the C-H bond is used for undergoing a cross-linking reaction with a first photosensitive group of the first photosensitive material; and
b is a positive integer taking a value greater than or equal to 2.

8. The light-emitting substrate according to claim 7, wherein the first photosensitive material includes the first photosensitive group; and
the first photosensitive group includes at least one of an azide group, a diazirine group and a diazo group.

9. The light-emitting substrate according to claim 3, wherein the material of the auxiliary layer is the same as the material of the hole transport layer, and the material of the auxiliary layer is a first material without cross-linking.

10. The light-emitting substrate according to claim 9, wherein the first material includes a hole transport material, or includes the hole transport material and a first photosensitive material.

11. The light-emitting substrate according to any one of claims 1 to 10, wherein a material of the light-emitting pattern contained in the first light-emitting device includes a first cross-linked quantum dot material, the first cross-linked quantum dot material being generated by cross-linking a first quantum dot material and a second photosensitive material under light radiation.

12. The light-emitting substrate according to claim 11, wherein the first cross-linked quantum dot material is selected from any one of structures shown in following general formula IV: 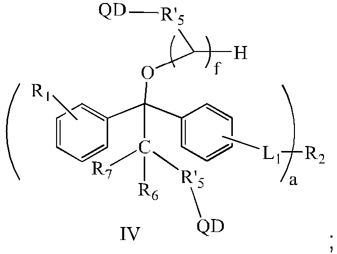
wherein L₁ is selected from any one of a single bond, an ester bond, an ether bond, and a thioether bond;
R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, wherein in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃;
R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain with an amino group, and a C1 to C40 carbon chain with a sulfhydryl group; and any one of the carboxyl group, the amino group, and the sulfhydryl group is connected to the quantum dot body by coordination; and
a is a positive integer taking a value greater than or equal to 1, and f is a positive integer taking a value greater than or equal to 1.

13. The light-emitting substrate according to claim 11 or 12, wherein the first cross-linked quantum dot material is selected from any one of structures shown in following general formula V: 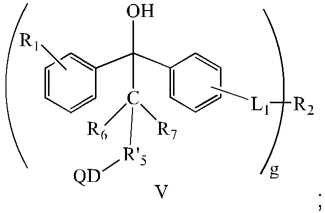
wherein L₁ is selected from any one of a single bond, an ester bond, an ether bond, and a thioether bond;
R₁, R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
R₂ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, wherein in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃;
R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination; and
g is a positive integer taking a value greater than or equal to 2.

14. The light-emitting substrate according to any one of claims 11 to 13, wherein the first cross-linked quantum dot material is selected from any one of structures shown in following general formula VIII: 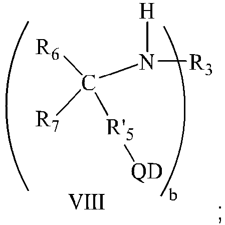
wherein QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, wherein in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃;
R₃ is selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination;
R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; and
b is a positive integer taking a value greater than or equal to 2.

15. The light-emitting substrate according to any one of claims 11 to 14, wherein the first cross-linked quantum dot material is selected from any one of structures shown in following general formula IX: 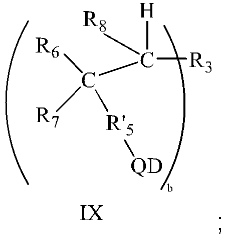
wherein QD represents any one quantum dot body, and the quantum dot body includes any one of a Group IIB-VIA quantum dot, a Group IIIA-VA quantum dot, a Group IVA-VIA quantum dot, a quantum dot with core-shell structure, and an ABX₃ type perovskite quantum dot, wherein in the ABX₃ type perovskite quantum dot, A is one or more of CH₃NH₃⁺, NH₂CH=NH₂, and Cs⁺, B is one or two of Pb²⁺ and Sn²⁺, X is one or more of Cl⁻, Br⁻, and I⁻, and ABX₃ type perovskite quantum dots include CH₃NH₃PbBr₃, CH₃NH₃PbCl₃, CH₃NH₃PbI₃, CsPbBr₃, CsPbCl₃, and CsPbl₃;
R₃ and R₈ are each independently selected from any one of a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40;
R'₅ is selected from a remaining structure of R₅ with one hydrogen removed from any one of a carboxyl group, an amino group, and a sulfhydryl group therein; R₅ is selected from any one of a C1 to C40 carbon chain with a carboxyl group, a C1 to C40 carbon chain an amino group, and a C1 to C40 carbon chain with a sulfhydryl group; and any one of the carboxyl group, the amino group, and the sulfhydryl group is connected to the quantum dot body by coordination;
R₆ and R₇ are the same or different, and are each independently selected from any one of a hydrogen bond, a saturated or unsaturated straight or branched alkyl group with C1 to C40, a cycloalkyl group with C3 to C40, a heterocycloalkyl group with C3 to C40, an aryl group with C6 to C40, and a heteroaryl group with C6 to C40; and
b is a positive integer taking a value greater than or equal to 2.

16. The light-emitting substrate according to any one of claims 11 to 15, wherein the first quantum dot material includes a quantum dot body and a first ligand material coordinated on the quantum dot body; and
the first ligand material contains a C-H bond, and/or the first ligand material contains a double bond.

17. The light-emitting substrate according to any one of claims 11 to 16, wherein the second photosensitive material includes a second photosensitive group, and the second photosensitive group includes a benzophenone group.

18. The light-emitting substrate according to any one of claims 1 to 17, wherein the plurality of light-emitting devices further include at least one second light-emitting device and at least one third light-emitting device, wherein
the at least one first light-emitting device is configured to emit light of first wavelength, the light of first wavelength being red light;
the at least one second light-emitting device is configured to emit light of second wavelength, the light of second wavelength being green light; and
the at least one third light-emitting device is configured to emit light of third wavelength, the light of third wavelength being blue light.

19. The light-emitting substrate according to any one of claims 1 to 18, wherein the light-emitting device includes multiple light-emitting patterns arranged in a stack, with a charge generation layer being provided between every two adjacent light-emitting patterns in the multiple light-emitting patterns.

20. A light-emitting apparatus, comprising the light-emitting substrate according to any one of claims 1 to 19; and
further comprising a driving circuit, for driving the light-emitting substrate to emit light.
